# EUROPÄISCHE PATENTSCHRIFT

(11) **EP 4 153 283 B1**
(45) Veröffentlichungstag und Bekanntmachung des Hinweises auf die Patenterteilung: **13.11.2024**
(21) Anmeldenummer: 21727108.9
(22) Anmeldetag: 14.05.2021
(51) Int. Cl.: A61M 16/04, A61B 1/00

(54) **BEATMUNGSVORRICHTUNG**
RESPIRATORY DEVICE
DISPOSITIF RESPIRATOIRE

(30) Priorität: 20.05.2020 DE 102020113654
(43) Veröffentlichungstag der Anmeldung: 29.03.2023
(73) Patentinhaber: Pfeifer, Jonas, 80337 München (DE)
(72) Erfinder: Pfeifer, Jonas, 80337 München (DE)
(74) Vertreter: Hannke Bittner & Partner mbB Regensburg
(86) Internationale Anmeldenummer: PCT/EP2021/062869
(87) Internationale Veröffentlichungsnummer: WO 2021/233784

(56) Entgegenhaltungen:
- DE-A1- 102018 123 562
- US-A- 4 722 335
- US-A1- 2010 313 896
- US-A1- 2015 011 828

## Beschreibung

Die Erfindung betrifft eine Beatmungsvorrichtung, insbesondere eine Trachealkanüle zum Einsetzen in ein Tracheostoma, umfassend ein Kanülenelement, welches ein Halselement und ein an dem Halselement angeordnetes Hohlkörperelement umfasst, wobei das Hohlkörperelement eine Innenwand aufweist, welche zumindest ein Innenlumen umgibt.

Für Patienten mit einem Luftröhrenschnitt, einer Tracheotomie oder Tracheostomie, stellt das Finden einer passenden Trachealkanüle, durch die die Beatmung des Patienten erfolgt, häufig eine große Herausforderung dar.

Es gibt mehrere Faktoren, die bei der Auswahl der Trachealkanüle zu berücksichtigen sind. Zum einen sind die individuelle Anatomie des Tracheostomas und der Trachea sowie deren Verhältnis zueinander zu bedenken. Wichtige Kenngrößen sind der Durchmesser, die Länge und der Verlauf des Tracheostomas, die Länge und der Verlauf der Trachea sowie der Winkel von Tracheostoma und Trachea zueinander. Diese Faktoren stellen für einen optimalen Sitz der Trachealkanüle bereits hohe Anforderungen an die Anpassbarkeit der Geometrie der Trachealkanüle. Weitere Auswahlfaktoren sind etwaige Sekretprobleme des Patienten, die eine austauschbare Innenseele oder eine Sekretabsaugemöglichkeit erfordern können oder die Kanüle verstopfen können. Des Weiteren ist die Möglichkeit einer Sprechfunktion zu bedenken.

Handelsübliche Trachealkanülen erfüllen nur wenige der oben genannten Anforderungen. Trachealkanülen werden häufig aus starrem Material wie Metall oder Kunststoff hergestellt. Diese Kanülen sind schlecht bis gar nicht an die individuelle Anatomie des Patienten anpassbar. Die führt nicht nur zu einem unangenehmen Tragegefühl für den Patienten, sondern birgt auch die Gefahr von Druckstellen oder Verletzungen des angrenzenden Gewebes. Die Anpassung an die Anatomie des Patienten erschöpft sich in der Bereitstellung der Kanüle in unterschiedlichen Größen.

Das Dokument DE 10 2018 123 562 A1 betrifft eine Beatmungsvorrichtung, insbesondere eine Trachealkanüle zum Einsetzen in ein Tracheostoma, umfassend einen Kanülenhals und ein flexibles Kanülenrohr, welches mit seinem distalen Ende am proximalen Ende des Kanülenhalses angeordnet ist, wobei der Kanülenhals und das Kanülenrohr einen fluidischen Strömungspfad umschließen, welcher vom distalen Ende des Kanülenhals zum proximalen Ende des Kanülenrohrs und in Gegenrichtung dazu von einem Fluid durchströmbar ist. Der Gegenstand ist dadurch gekennzeichnet, dass das Kanülenrohr zumindest ein Innenlumen und zumindest ein Außenlumen aufweist und das Innenlumen innerhalb des Außenlumen angeordnet ist, insbesondere ähnlich einer Doppelwandkonstruktion, wobei der Zwischenraum zwischen dem Innenlumen und dem Außenlumen entlang der Längsachse des Kanülenrohrs in zumindest zwei Luftkammern unterteilt ist, welche vom distalen Ende des Kanülenhals aus durch ein Luftkammerventil befüll- und/oder entleerbar sind. Das Dokument US 4 722 335 A umfasst als Gegenstand eine Trachealkanüle oder dergleichen mit zwei sich überlappenden Segmenten die zusammen eine Röhre bilden. Zwischen dem innenliegenden Segment und dem außenliegenden Segment sind gleitfähige Dehnungsfugen vorhanden, die die Segmente miteinander verbinden und zulassen, dass sich die Röhre in deren Durchmesser vergrößern kann. Dazu befindet sich im äußeren Segment eine entlang der Längsrichtung verlaufende Zuleitung, die mittels einer Spritze über die Öffnung mit Luft befüllt werden kann, wobei die Zuleitung mit Kathetern verbunden ist. Werden die Katheter mit Luft bedruckt, biegen diese sich auf. Das innere Segment gleitet in Folge entlang der Dehnungsfugen, wobei der Durchmesser der Röhre vergrößert wird. Das Dokument US 2010/313896 A1 zeigt einen Endotrachealtubus mit einer Doppelwandstruktur, die eine Innenwandung und eine Außenwandung umfasst, wobei Luft zwischen die Wandungen und eingebracht werden kann, um diesen von einem flexiblen in einen formstabilen Zustand zu bringen. Das Dokument US 2015/011828 A1 beschreibt eine Trachealkanüle. Optional kann die Trachealkanüle eine expandierbare Membran umfassen, die das Rohrelement umgibt. Das Rohrelement weist in Längsrichtung eine Öffnung auf, wobei die Enden ausgebildet werden, die sich ähnlich einer Nut- und Federverbindung in einem "H"-förmigen Verbindungselement befinden. In dem Verbindungselement werden Kavitäten ausgebildet, in denen sich befüllbare Ballons befinden. Durch das Befüllen der Ballons werden die Kavitäten gefüllt und die Enden nach außen aus dem Verbindungselement gedrückt, sodass sich der Durchmesser des Rohrelements vergrößert.

Angenehmer zu tragende Trachealkanülen aus weichem Material, wie z.B. Silikon, sind zwar auf dem Markt verfügbar, sind jedoch in der Regel nicht mit einer Sprechfunktion kombinierbar, da auf Grund der Weichheit des Materials höhere Materialstärken und damit auch höhere Außendurchmesser der Kanülen nötig wären, um die notwendige Knickstabilität zu erreichen.

Diese höheren Materialstärken sind häufig mit der Anatomie des Patienten nicht oder nur schlecht vereinbar. Kanülen ohne Sprechfunktion wirken sich in der Regel jedoch sehr negativ auf die Rekonvaleszenz des Patienten aus und erhöhen den Leidensdruck beim Patienten stark, da bereits ein zeitweiser Verlust der Sprache eine extreme Beeinträchtigung darstellt.

Die Sprechfunktion wird bei den starren Kanülen häufig durch eine Fensterung im Bereich des Übergangs zwischen Kanülenhals und Kanülenelement realisiert, wobei die Kanüle über eine austauschbare Innenseele mit und ohne Fensterung verfügt, sodass zwischen normalem Beatmen und Sprechen gewechselt werden kann. Das Kanülenelement wird oftmals auch als Kanülenrohr bezeichnet. Durch diese Fensterung kann bei der Ausatmung Luft über die Stimmbänder in die oberen Atemwege gelangen, sodass dem Patienten ein Luftstrom für die Phonation zur Verfügung steht. Neben dem Austausch der Innenseele muss häufig ein Sprechventil aufgesetzt werden, welches ein Ausatmen über die Kanüle verhindert und so den Luftstrom über die oberen Atemwege erhöht. In Summe besitzen derartige Kanülen ein großes Potenzial für Fehlbedienungen durch das Pflegepersonal, ein erhöhtes Infektionsrisiko, geringe Flexibilität und haben bedingt durch die Innenseelen, große Durchmesser.

Aufgabe der vorliegenden Erfindung ist es daher, eine Trachealkanüle bereit zu stellen, die eine verbesserte Anpassbarkeit an die individuelle Anatomie des jeweiligen Patienten ermöglicht und gleichzeitig eine Sprechfunktion aufweist, sodass die Kanüle bei möglichst vielen Patienten mit verschiedenen medizinischen Indikationen verwendbar ist.

Diese Aufgabe wird gelöst durch eine Beatmungsvorrichtung gemäß Anspruch 1. Bevorzugte Ausführungsformen werden in den Unteransprüchen angeführt.

Es wird eine Beatmungsvorrichtung, insbesondere eine Trachealkanüle zum Einsetzen in ein Tracheostoma bereitgestellt, umfassend ein Kanülenelement, welches ein Halselement und ein an dem Halselement angeordnetes Hohlkörperelement umfasst, wobei das Hohlkörperelement eine Innenwandung aufweist, welche zumindest ein Innenlumen umgibt, wobei die Innenwandung von einer Außenwandung umgeben ist, wobei zwischen der Innenwandung und der Außenwandung ein Zwischenlumen definiert ist, in welchem zumindest ein vorzugsweise rohrartig ausgebildeter Stabilisierungskanal angeordnet ist, wobei in einem ersten Zustand das Hohlkörperelement in einem flexiblen Zustand ist, wobei durch Zuführen einer vorbestimmten Menge an Stabilisierungsfluid in die Stabilisierungskanäle das Hohlkörperelement in einen zweiten formstabilen Zustand bringbar ist, wobei zwischen der Innenwandung und der Außenwandung zumindest eine Zwischenschicht vorgesehen ist.

Das Halselement kann auch als Kanülenhals bezeichnet werden und umfasst vorzugsweise ein distales Ende und ein proximales Ende. An dem proximalen Ende ist dabei ein distales Ende des Hohlkörperelements angeordnet. Das Kanülenelement, beziehungsweise das Halselement und das Hohlkörperelement umschließen einen fluidischen Strömungspfad, welcher von dem distalen Ende des Kanülenelements, beziehungsweise von dem distalen Ende des Halselements zu dem proximalen Ende des Kanülenelements, beziehungsweise dem proximalen Ende des Hohlkörperelements sich erstreckt. Der Strömungspfad kann sowohl in beiden Richtungen von einem Fluid durchströmt werden. Vorzugsweise ist dieses Fluid Luft oder Atemluft.

Das Hohlkörperelement ist vorzugsweise ähnlich einer Doppelwandkonstruktion ausgebildet.

Ferner ist das Hohlkörperelement vorzugsweise ein länglicher Hohlkörper, dessen Länge in der Regel wesentlich größer als sein Durchmesser ist. Das Hohlkörperelement könnte vorteilhafterweise auch als Rohrelement bezeichnet werden. In dem Zwischenlumen ist vorzugsweise eine Mehrzahl an Stabilisierungskanälen angeordnet. In dem ersten Zustand ist das Hohlkörperelement in einem flexiblen ersten Zustand. In einem zweiten Zustand ist das Hohlkörperelement in einem formstabilen oder starren Zustand. Hierunter ist zu verstehen, dass das Hohlkörperelement eine vorbestimmte Form aufweist, welche unter einer Druck- und/oder Kraftbeaufschlagung stabil bleibt. Eine solche Druck- und/oder Kraftbeaufschlagung soll in einem Bereich liegen, welcher die bei einer üblichen Verwendung einer Trachealkanüle auftretenden Drücke und/oder Kräfte umfasst. In dem flexiblen ersten Zustand kann bereits eine geringe Druck- und/oder Kraftbeaufschlagung die Form des Hohlkörperelements verändern. Ferner ist das Hohlkörperelement im ersten Zustand zumindest teilweise zusammenfaltbar und/oder zusammendrückbar. Im zweiten Zustand wird die vorteilhafte rohrförmige Form der Innenwandung durch die Stabilisierungskanäle stabilisiert. Durch diese Stabilisierung kann vorzugsweise eine sehr viel dünnere Materialstärke für die Innenwandung und/oder die Außenwandung verwendet werden, als wenn das Material selbst die nötige Stabilität erzeugen müsste. Damit kann eine im zweiten Zustand sehr stabile und dabei im ersten Zustand sehr flexible Trachealkanüle bereitgestellt werden. Insbesondere nimmt eine solche in der Trachea angeordnete und sich im ersten Zustand befindliche Trachealkanüle ein so geringes Volumen ein, dass beim Ausatmen Luft, an der Trachealkanüle vorbei in Richtung der Stimmbänder, in die oberen Atemwege leitbar ist. Damit weist die erfindungsgemäße Trachealkanüle im ersten Zustand eine Sprechfunktion auf.

Im ersten Zustand des Hohlkörperelements kann in dem zumindest einem Stabilisierungskanal bereits eine bestimmte Menge an Stabilisierungsfluid, beispielsweise Luft, enthalten sein. Diese Menge ist jedoch nicht ausreichend, um einen im Wesentlichen starren oder formstabilen Zustand zu verursachen. Der starre oder formstabile zweite Zustand wird durch ein Einführen von einer vorbestimmten Menge an Stabilisierungsfluid in S den zumindest einen Stabilisierungskanal erreicht. Das Stabilitätsfluid übt auf die Innenflächen des zumindest einen Stabilitätskanals entsprechende Kräfte aus. In dem ersten Zustand sind diese entsprechenden Kräfte klein genug, damit das Hohlkörperelement in einem flexiblen Zustand verbleibt. Wird nun eine vorbestimmte Menge an Stabilisierungsfluid in den zumindest einen Stabilisierungskanal eingeführt, wird die Teilchenzahl des Fluids erhöht. Dementsprechend wird der Druck in dem zumindest einem Stabilisierungskanal, beziehungsweise die Kraft, auf die Innenflächen der Stabilisierungskanäle erhöht. Durch diesen Innendruck wird entsprechender Druck und/oder Kraftbeaufschlagungen auf das Hohlkörperelement entgegengewirkt, wodurch sich eine Formstabilität des Hohlkörperelements ergibt. Vorzugsweise ist ein Grenzwert der Menge an Stabilisierungsfluid, beziehungsweise ein Grenzwert des Innendrucks, in dem zumindest einem Stabilisierungskanal vorgesehen. Bei einem Überschreiten dieses Grenzwerts findet vorteilhafterweise ein Übergang von dem ersten Zustand zu dem zweiten Zustand statt.

In dem ersten flexiblen Zustand bleibt jedoch ein Mindestmaß an Stabilität der Trachealkanüle erhalten. Insbesondere kann in dem ersten Zustand noch Atemluft durch die Trachealkanüle in den Patienten hineinfließen. Bevorzugt ist der Rückfluss der Ausatemluft durch die Trachealkanüle im ersten Zustand zumindest reduziert, sodass die Ausatemluft verstärkt in Richtung der Stimmbänder und oberen Atemwege gelenkt wird und eine Phonation erleichtert wird. Somit ist die Verwendung eines gesonderten Sprechventils oder Sprechaufsatzes nicht mehr notwendig. Insbesondere ist bevorzugt, dass der Rückfluss der Ausatemluft fast vollständig über die oberen Atemwege und damit nicht durch die Trachealkanüle erfolgt. Dabei ist es denkbar, dass der freie Innendurchmesser der Trachealkanüle durch Beaufschlagung der Stabilisierungskanäle mit einem Unterdruck weiter reduzierbar ist. Dadurch ist auch der Platzbedarf der Kanüle in der Trachea weiter reduzierbar, sodass Sprechversuche auch bei anatomisch beengten Verhältnissen in der Trachea möglich sind.

Nach einer weiteren bevorzugten Ausführungsform ist der zumindest eine Stabilisierungskanal bei Einführung der vorbestimmten Menge an Stabilisierungsfluid und dem sich daraus ergebenden Innendruck zumindest abschnittsweise bevorzugt nicht vollständig deformierbar.

Nach einer bevorzugten Ausführungsform sind an dem distalen Ende des Halselements ein Kanülenkonnektor und ein Kanülenschild angeordnet. Ein solches Kanülenschild dient zur Befestigung der Kanüle am Hals.

Vorzugsweise ist das Halselement mit dem Kanülenschild durch eine Gelenkvorrichtung verbunden, sodass das Halselement aus der Flächennormalen des Kanülenschilds heraus in alle Raumrichtungen kippbar ist. Bevorzugt ist das Halselement gegenüber dem Kanülenschild bis zu 45°, besonders bevorzugt bis 30°, in alle Raumrichtungen kippbar. Bevorzugt ist das Kanülenschild in seiner flächigen Ausdehnung senkrecht zur Längsachse des Halselements angeordnet und weist mittig eine Ausnehmung auf, sodass der Strömungspfad aus dem Halselement heraus und/oder in das Halselement hinein durch die Ausnehmung verläuft. Das Kanülenschild liegt bei am Patienten angeordneter Trachealkanüle auf der vorderen Außenseite des Halses rund um das Tracheostoma herum auf und stabilisiert den Kanülenhals im Tracheostoma. Insbesondere verhindert das Kanülenschild ein zu weites Hineinrutschen oder Herausrutschen der Trachealkanüle in das Tracheostoma. Auch liegt das Kanülenschild durch die Gelenkvorrichtung unabhängig vom Winkel zwischen Tracheostomaschaft und äußerer Halsoberfläche flächig auf dieser auf und stabilisiert die Trachealkanüle. Beispielsweise kann an dem Kanülenschild ein Kanülentrageband angebracht sein, welches um den Hals des Patienten gelegt wird, wodurch die Trachealkanüle fixiert wird. Derartige Tragebänder können gepolstert oder ungepolstert sein. Ferner können die Kanülentragebänder ein-, oder mehrteilig sein und mit unterschiedlichen Befestigungsmechanismen an dem Kanülenschild befestigt sein. An dem vorteilhaften Kanülenkonnektor kann beispielsweise ein Beatmungsschlauch oder ein Aufsatz angebracht werden. Ein solcher Aufsatz kann beispielsweise ein Wärmefeuchtigkeitsaustauscher sein. Diese Wärmefeuchtigkeitsaustauscher ersetzen vorteilhafterweise die fehlende Funktion der Nase (Befeuchten, Erwärmen und Filtern der Luft) und werden demnach auch als "Feuchte Nasen" bezeichnet. Der Wärmefeuchtigkeitsaustauscher besteht vorteilhafterweise aus einem Kunststoffgehäuse und einem Filterkern, der aus Schaumstoff oder Papier gefertigt ist. Bei der Ausatmung wird die in der Atemluft befindliche Feuchtigkeit im Filter gespeichert und bei der nächsten Einatmung wieder an die Atemluft abgegeben. So wird vermieden, dass die Schleimhäute der Luftröhre austrocknen und dort befindlicher Schleim sich verfestigt und die Atemwege einengt. Darüber hinaus wird die Atemluft gefiltert und verhindert die Einatmung von Fremdkörpern und Staubpartikel.

Nach einer weiteren bevorzugten Ausführungsform ist das Stabilisierungsfluid über zumindest eine Zugangseinrichtung dem zumindest einem Stabilisierungskanal zuführbar und/oder aus dem zumindest einem Stabilisierungskanal abführbar. Denkbar wäre somit, dass zumindest eine oder lediglich eine Zugangseinrichtung dafür vorgesehen ist, den zumindest einen Stabilisierungskanal mit dem Stabilisierungsfluid zu befüllen. Denkbar wäre weiterhin, dass zumindest eine oder lediglich eine Zugangseinrichtung dafür vorgesehen ist, den zumindest einen Stabilisierungskanal zu entleeren. Denkbar wäre jedoch auch, dass zumindest eine oder lediglich eine Zugangseinrichtung vorgesehen ist, mittels welcher ein Befüllen als auch ein Entleeren des zumindest einen Stabilisierungskanals möglich ist. Vorzugsweise besteht an einem distalen Ende des Hohlkörperelements eine fluidische Verbindung zwischen der zumindest einen Zugangseinrichtung und dem zumindest einem Stabilisierungskanal, beziehungsweise zumindest einer Teilmenge an Stabilisierungskanälen. Vorzugsweise ist das Stabilisierungsfluid Luft. Denkbar wäre jedoch auch die Verwendung weiterer geeigneter flüssiger oder gasförmiger Fluide.

Nach einer weiteren vorteilhaften Ausführungsform umfasst die zumindest eine Zugangseinrichtung zumindest eine aus der Beatmungsvorrichtung führende Verbindungseinrichtung. Eine solche Verbindungseinrichtung kann beispielsweise ein Schlauchelement oder ein Rohrelement sein. Vorzugsweise tritt die zumindest eine Verbindungseinrichtung durch eine Außenwandung des Halselements. Dieser Austritt kann an oder in der Nähe des proximalen Endes des Halselements sein. Dementsprechend würde der Austritt in der Nähe des Übergangs zwischen dem Halselement und dem Hohlkörperelement sein. Alternativ oder kumulativ kann der Austritt der zumindest einen Verbindungseinrichtung aus dem Halselement an oder in der Nähe des distalen Endes des Halselements sein. Dementsprechend würde der Austritt in der Nähe des Übergangs zu dem Kanülenkonnektor vorgesehen sein. Vorzugsweise umfasst die zumindest eine Zugangseinrichtung eine Ventileinrichtung. Vorteilhafterweise ist durch die Ventileinrichtung der Zugang zu den Stabilisierungskanälen verschließbar. Vorteilhafterweise ist durch die Ventileinrichtung der Innendruck in den Stabilisierungskanälen modellierbar, beziehungsweise regulierbar.

Nach einer bevorzugten Ausführungsform weist das Hohlkörperelement, beziehungsweise das Kanülenelement, in dem zweiten Zustand einen gekrümmten Verlauf auf. Vorteilhafterweise weist das Hohlkörperelement einen Krümmungswinkel α auf, welcher in einem Bereich von 90°-120° liegt. In der Regel wird der Krümmungswinkel vom individuellen Verlauf der Luftröhre abhängig gemacht. Bei Erwachsenen wird jedoch meist ein Krümmungswinkel von 90° gewählt, bei Kindern einer im Bereich von 105°-120°. Vorzugsweise passt sich das Kanülenelement, beziehungsweise das Hohlkörperelement, beziehungsweise der Krümmungswinkel, dieser während des Einführens des Stabilisierungsfluids in die Stabilisierungskanäle an den Verlauf der Trachea an. So ist die Trachealkanüle auch bei einem schlecht angelegten Tracheostoma verwendbar. Bei handelsüblichen Trachealkanülen muss die Positionierung der Kanüle nach dem Einsetzen in das Tracheostoma in der Regel endoskopisch kontrolliert werden. Durch die selbstzentrierende Wirkung bei gleichzeitiger optimaler Anpassung an den Tracheaverlauf, z.B. wenn dieser nicht vollständig gerade ist, ist eine endoskopische Lagekontrolle bei der erfindungsgemäßen Trachealkanüle nicht notwendig. Dementsprechend ist die Menge an Stabilisierungsfluid in den Stabilisierungskanälen, beziehungsweise der Innendruck oberhalb des entsprechenden Grenzwerts variierbar. Dies kann vorteilhafterweise durch die zumindest eine Zugangseinrichtung, beziehungsweise durch die Ventileinrichtung der zumindest einen Zugangseinrichtung erfolgen. Hierdurch kann eine optimale Anpassung an die Trachea vorgenommen werden. Der gekrümmte Verlauf des Hohlkörperelements kann durch gezielte Auswahl von Materialdicken der Innen- und/oder Außenwandungen erfolgen. Ferner kann die Krümmung durch entsprechenden Längenunterschiede der Innenwandung und oder Außenwandung bestimmt werden. Ferner kann durch Längenunterschiede und/oder Orientierungsunterschiede der Stabilisierungskanäle die Krümmung begünstigt werden.

Nach einer weiteren vorteilhaften Ausführungsform weist der zumindest eine Stabilisierungskanal, beziehungsweise zumindest eine Teilmenge der Stabilisierungskanäle eine Erstreckung auf, welche im Wesentlichen von einem distalen Ende des Hohlkörperelements ausgeht. Demnach beginnt der zumindest eine Stabilisierungskanal, beziehungsweise beginnen die zu dieser Teilmenge gehörigen der Stabilisierungskanäle im Wesentlichen, d.h. an dem distalen Ende des Hohlkörperelements oder in unmittelbarer Nähe des distalen Endes des Hohlkörperelements. Bei Ausführungsformen, in welchen eine Teilmenge an Stabilisierungskanälen vorhanden ist, welche nicht von dem distalen Ende des Hohlkörperelements ausgeht, ist eine vorteilhafte fluidische Verbindung zu einer solchen Teilmenge vorgesehen, welche einen Ausgang am distalen Ende des Hohlkörperelements hat. Hierdurch wird gewährleistet, dass alle Stabilisierungskanäle eine fluidische Verbindung zu der vorteilhaften Zugangseinrichtung aufweisen und somit mit Stabilisierungsfluid befüllbar, beziehungsweise entleerbar sind.

Vorzugsweise erstreckt sich der zumindest eine Stabilisierungskanal, beziehungsweise zumindest eine Teilmenge der Stabilisierungskanäle im Wesentlichen bis zu einem proximalen Ende des Hohlkörperelements. Demnach endet der zumindest eine Stabilisierungskanal, beziehungsweise zumindest eine Teilmenge der Stabilisierungskanäle, an dem proximalen Ende des Hohlkörperelements oder in unmittelbarer Nähe zu dem proximalen Ende des Hohlkörperelement. Vorzugsweise erstreckt sich der zumindest eine Stabilisierungskanal, beziehungsweise zumindest eine Teilmenge der Stabilisierungskanäle, sich im Wesentlichen entlang einer Längsrichtung des Hohlkörperelements. Bevorzugt ist diese Erstreckung im Wesentlichen ausgehend von dem distalen Ende des Hohlkörperelements, im Wesentlichen entlang einer Längsrichtung des Hohlkörperelements bis im Wesentlichen zu dem proximalen Ende des Hohlkörperelements. Vorteilhafterweise erstreckt sich der zumindest eine Stabilisierungskanal, beziehungsweise zumindest eine Teilmenge der Stabilisierungskanäle im Wesentlichen geradlinig oder helixartig entlang der Längsrichtung des Hohlkörperelements. Denkbar wären jedoch auch noch anderweitige Erstreckungen. Die Stabilisierungskanäle können sich entlang einer lateralen, vertikalen oder horizontalen Richtung des Hohlkörperelements erstrecken. Denkbar wäre, dass ein Stabilisierungskanal vollständig oder zumindest sich abschnittsweise entlang der Umfangsrichtung des Hohlkörperelements erstreckt. Vorzugsweise sind entlang der Längsrichtung des Hohlkörperelements dann eine Mehrzahl solcher sich in Umfangsrichtung erstreckender Stabilisierungskanäle angeordnet. Die einzelnen Stabilisierungskanäle weisen jedoch jeweils eine fluidische Verbindung zueinander auf. Bevorzugt mündet zumindest eine Teilmenge der Stabilisierungskanäle in einem proximalen Verschluss. Vorzugsweise ist die Gesamtanordnung der Stabilisierungskanäle, beziehungsweise der Teilmengen an Stabilisierungskanälen derart ausgebildet, dass am Ende eines Strömungspfads des Stabilisierungsfluids durch die Strömungskanäle ein Verschluss oder ein Strömungswiderstand vorgesehen ist. Hierdurch wird vorteilhafterweise ein Aufbau eines Innendrucks in den Stabilisierungskanälen gewährleistet.

Nach einer weiteren vorteilhaften Ausführungsform sind zwischen den Stabilisierungskanälen Zwischenräume vorgesehen. Vorteilhafterweise werden diese Zwischenräume nicht mit dem Stabilisierungsfluid befüllt. Vorzugsweise weisen die Stabilisierungskanäle einen runden Querschnitt oder einen polygonartigen Querschnitt auf. Ein polygonartiger Querschnitt kann beispielsweise ein quadratischer, rechteckiger, dreieckiger, fünfeckiger Querschnitt sein. Die Anordnung der Stabilisierungskanäle kann auch ein Bienenwabenmuster aufweisen. Denkbar ist auch, dass die Gesamtheit der Stabilisierungskanäle Teilmengen mit unterschiedlichen Querschnitten aufweist. Vorzugsweise weist die Gesamtheit der Stabilisierungskanäle Teilmengen mit unterschiedlichen Durchmessern auf.

Nach einer weiteren vorteilhaften Ausführungsform ist zwischen der Innenwandung und der Außenwandung zumindest ein Stegelement angeordnet. Vorzugsweise erstreckt sich das zumindest eine Stegelement im Wesentlichen entlang einer radialen Richtung des Hohlkörperelements. Bevorzugt ist der zumindest eine Stabilisierungskanal durch einen Abschnitt der Innenwandung, einen Abschnitt der Außenwandung und dem zumindest einem Stegelement gebildet. Vorteilhafterweise ist eine Vielzahl an voneinander beabstandeten Stegelementen vorgesehen, welche den Verlauf der Stabilisierungskanäle definieren. Vorzugsweise ist eine Vielzahl von Stegelementen vorgesehen, welche entlang der Umfangsrichtung des Hohlkörperelements angeordnet sind. Vorzugsweise erstrecken sich die Stegelemente ausgehend von dem distalen Ende des Hohlkörperelements geradlinig oder helixartig entlang der Längsrichtung des Hohlkörperelements zu dem proximalen Ende des Hohlkörperelements. Das zumindest eine Stegelement kann aus dem gleichen Material wie die Innenwandung und/oder die Außenwandung bestehen. Denkbar wäre jedoch auch, dass das zumindest eine Stegelement aus einem unterschiedlichen Material wie die Innenwandung und/oder die Außenwandung besteht. Denkbar wäre auch, dass einzelne Stegelemente aus unterschiedlichen Materialien bestehen. Vorteilhafterweise besteht das zumindest eine Stegelement aus einem elastischen Material. Hierdurch wird ein vorteilhaftes Einfalten des Hohlkörperelements in dem ersten Zustand begünstigt. Die Orientierung dieser durch die Stegelemente begrenzten Stabilisierungskanäle ist analog zu den Ausführungsformen der generell beschriebenen Orientierung der Stabilisierungskanäle. Dementsprechend können auch fluidische Verbindungen zwischen den Stabilisierungskanälen vorgesehen sein.

Nach einer weiteren vorteilhaften Ausführungsform ist zwischen der Innenwandung und der Außenwandung zumindest ein Röhrenelement vorgesehen, welches den zumindest einen Stabilisierungskanal bildet. Vorteilhafterweise ist zwischen der Innenwandung und der Außenwandung eine Mehrzahl an Röhrenelementen vorgesehen, welche zumindest eine Teilmenge der Stabilisierungskanäle bilden. Vorteilhafterweise umschließt das zumindest eine Röhrenelement den zumindest einen Stabilisierungskanal vollständig. Demnach würden weder ein Abschnitt der Innenwandung noch ein Abschnitt der Außenwandung den zumindest einen Stabilisierungskanal begrenzen. Denkbar wäre jedoch auch, dass die Röhrenelemente nicht vollständig geschlossen sind und zusätzlich ein Abschnitt der Innenwandung und/oder ein Abschnitt der Außenwandung den zumindest einen Stabilisierungskanal begrenzen. Das zumindest eine Röhrenelement kann vorteilhafterweise mit der Innenwandung und/oder der Außenwandung verbunden sein. Eine solche Verbindung kann eine stoffschlüssige und/oder eine kraftschlüssige und/oder eine formschlüssige Verbindung sein. Die Orientierung des zumindest einen Röhrenelement ist analog zu den Ausführungsformen der generell beschriebenen Orientierung der Stabilisierungskanäle. Dementsprechend können auch fluidische Verbindungen zwischen Röhrenelementen vorgesehen sein. Denkbar wäre auch, dass sowohl Stabilisierungskanäle verwendet werden, welche wie oben beschrieben durch Stegelemente gegrenzt sind als auch Stabilisierungskanäle, welche durch Röhrenelemente ausgebildet sind. Die Röhrenelemente können aus dem gleichen Material wie die Innenwandung und/oder die Außenwandung bestehen. Denkbar wäre jedoch auch, dass die Röhrenelemente aus unterschiedlichen Materialien wie die Innenwandung und/oder die Außenwandung bestehen.

Vorzugsweise ist unabhängig von der individuellen Orientierung und der individuellen Ausgestaltung der Stabilisierungskanäle ein Strömungspfad des Stabilisierungsfluids durch die Stabilisierungskanäle definiert, welcher sich im Wesentlichen vom distalen Ende des Hohlkörperelements bis im Wesentlichen sich in dem proximalen Ende des Hohlkörperelements erstreckt.

Die Zwischenschicht zwischen der Innenwandung und der Außenwandung kann sich entlang der Längsrichtung und/oder entlang der Umfangsrichtung des Hohlkörperelements und/oder entlang einer radialen Richtung erstrecken. Diese Erstreckung kann lediglich abschnittsweise oder entlang der gesamten Längsrichtung und/oder entlang der gesamten Umfangsrichtung des Hohlkörperelements sein. Die zumindest eine Zwischenschicht kann zur Stabilisierung bestimmter Abschnitte des Hohlkörperelements oder zur Stabilisierung des gesamten Hohlkörperelements dienen. Ferner kann die Formgebung, beispielsweise die Krümmung in dem zweiten Zustand, durch ein Vorsehen zumindest einer Zwischenschicht vorgegeben werden. Vorteilhafterweise ist die zumindest eine Zwischenschicht mit der Innenwandung und/oder der Außenwandung verbunden. Die Verbindung kann punktuell oder entlang der gesamten Erstreckung sein. Die Verbindung kann eine stoffschlüssige und/oder eine kraftschlüssige und/oder eine formschlüssige Verbindung sein. Denkbar wäre auch, dass die Zwischenschicht freiliegen zwischen der Innenwandung und der Außenwandung angeordnet ist.

Bevorzugt werden für die Innenwandung und die Außenwandung thermoplastische Materialien, Silikon oder Verbundwerkstoffe verwendet. Es ist jedoch auch jedes andere dem Fachmann bekannte und für den Einsatzzweck geeignete Material denkbar. Die Materialien sind derart gewählt, dass im ersten Zustand das Hohlkörperelement flexibel, beziehungsweise eingefaltet ist. Die Stegelemente oder die Hohlkörperelemente können ebenso aus diesen Materialien hergestellt werden. Bevorzugt weist die Innenwandung eine höhere Materialstärke auf als die Außenwandung. Hierdurch weist die Innenwandung im zweiten Zustand des Hohlkörperelements eine im Wesentlichen glatte Innenoberfläche auf. Die Innenfläche der Innenwandung umschließt den Stömungspfad des Fluids und ist im zweiten Zustand vorzugsweise stabil, und/oder flach und/oder durchgehend gerade, d.h. es sind keine Einwölbungen oder Auswölbungen vorhanden. Demnach treten keine Verwirbelungen des Atemgases im Strömungspfad auf. Die Außenwandung weist bevorzugt eine geringere Materialstärke auf als die Innenwandung. Im zweiten Zustand nimmt das Hohlkörperelement lediglich seine nicht eingefaltete Ausbildung, beziehungsweise Form ein. Die Außenwandung wird vorzugsweise im zweiten Zustand nicht wesentlich expandiert, wie es beispielsweise bei einem Aufblasvorgang erfolgen würde. Weiterhin ist es denkbar, dass die Innenwandung und die Außenwandung aus unterschiedlichen Materialien hergestellt sind. Vorzugsweise ist es weiterhin denkbar, dass die Wandstärken beider Wandungen gleich sind.

Vorzugsweise umfasst das Halselement ein rohrartiges Innenelement. Dieses Innenelement kann vorteilhafterweise an seiner Außenseite vollständig oder abschnittsweise von der Außenwandung des Hohlkörperelements umgeben sein. Vorteilhafterweise kann dieses Innenelement an seiner Innenseite vollständig oder abschnittsweise von der Innenwandung des Hohlkörperelements umgeben sein. Das Innenelement kann vorzugsweise formstabil oder starr ausgebildet sein. Bevorzugt kann das Innenelement soweit flexibel ausgebildet sein, dass es sich in beliebige Richtungen leicht dehnen oder biegen lässt. Hierdurch kann eine leichtere Anpassung an die individuellen Anforderungen des Patienten gewährleistet werden. Vorzugsweise ist das Innenelement jedoch derart stabil, dass es durch einen externen Druck in sich stabil bleibt und den Strömungspfad des Fluids nicht verengt oder gar blockiert. Für die Anpassung an unterschiedliche Tracheostomadicken werden verschiedene Kanülengrößen mit verschiedenen Durchmessern des Kanülenhalses angeboten.

Gemäß zumindest einer weiteren Ausführungsform ist das Halselement in seiner Länge entlang des Strömungspfads verstellbar. Dies ist insbesondere vorteilhaft, da so eine individuelle Anpassung der Trachealkanüle an verschiedene Tracheostomaschaftlängen möglich ist. Bei einem zu kurzen Halselement würde das Hohlkörperelement in unangenehmer Weise an der ventralen Innenwand der Trachea anliegen, während es bei einem zu langen Halselement in unangenehmer Weise an der dorsalen Innenwand der Trachea anliegen würde. Da ein wie auch immer geartetes Anliegen der Kanüle an der Tracheainnenwand von Schleimhautreizungen bis hin zu Verletzungen oder sogar Nekrosen führen kann, ist eine optimale Anpassung notwendig, was durch die Längenverstellbarkeit des Halselements gewährleistet wird. Gemäß zumindest einer weiteren Ausführungsform weist das Halselement zwei ineinander angeordnete Hülsen auf, wobei die innere Hülse mit dem Innenlumen verbunden ist und die äußere Hülse mit dem Außenlumen verbunden ist und die Hülsen relativ zueinander entlang ihrer Längsachse gegeneinander verschiebbar sind. Gemäß zumindest einer weiteren Ausführungsform ist die innere Hülse gegenüber der äußeren Hülse in zumindest zwei entlang der Hülsenlängsachse gegeneinander verschobenen Positionen arretierbar.

Gemäß zumindest einer weiteren Ausführungsform sind die Hülsen durch eine zueinander relative Drehbewegung entlang ihres Umfangs in einer Drehrichtung voneinander lösbar und damit in Längsrichtung gegeneinander verschiebbar und durch eine relative Drehbewegung in entgegengesetzter Drehrichtung aneinander arretierbar. Dabei ist es vorteilhaft, wenn die innere Hülse auf der äußeren Hülsenfläche ein parallel zur Flächennormale der Hülsenoberfläche angeordnetes Dornelement aufweist und die äußere Hülse eine längliche Ausnehmung in der Hülsenwand parallel zur Hülsenlängsachse aufweist, in welcher das Dornelement der inneren Hülse führbar ist und die längliche Ausnehmung an zumindest einer Längsseite entlang der Hülsenlängsachse versetzt angeordnete Ausbuchtungen aufweist, in welchen das Dornelement arretierbar ist. Durch einen solchen Klick-Dreh-Verschluss ist die Anpassung der Länge des Halselements in besonders einfacher Weise durchführbar.

Vorzugsweise weist die Trachealkanüle eine Länge zwischen 39 mm und 135 mm auf. Die Länge der Trachealkanüle kann dabei auf die individuellen Gegebenheiten angepasst werden. Der Außendurchmesser der Trachealkanüle, beziehungsweise des Hohlkörperelements im zweiten Zustand liegt zwischen 4 mm - 18 mm. Der radiale Abstand zwischen der Innenwandung und der Außenwandung liegt in einem Bereich zwischen 0,5mm und 5mm. Der Innendruck in den Strömungskanälen im zweiten Zustand liegt zwischen 10 mbar und 70 mbar, bevorzugt zwischen 15 mbar und 50 mbar, weiter bevorzugt zwischen 25 mbar und 35 mbar.

Gemäß zumindest einer weiteren bevorzugten Ausführungsform ist an einem proximalen Ende des Hohlkörperelements ein Cuff angeordnet. Vorzugsweise ist der Cuff zu einer vorbestimmten Größe expandierbar. Bevorzugt erfolgt die Expansion durch Zuführung eines Expansionsfluids. Vorteilhafterweise ist das Expansionsfluid Luft oder ein anderweitiges gasförmiges oder flüssiges Fluid. Der Cuff ist vorzugsweise mit dem Expansionsfluid befüll- und/oder entleerbar. Vorzugsweise besteht der Cuff aus einem elastischen Material, welches eine entsprechende Expansion des Cuffs ermöglicht. Der Cuff dient dazu, die unteren Atemwege vor Sekreten aus den oberen Atemwegen zu schützen. Der Cuff dient auch dazu, den Weg zu den oberen Atemwegen zu verschließen, damit die Luft nur durch die Trachealkanüle fließt. Der Cuff dichtet daher im aufgeblasenen Zustand die Trachea ab. Da der konstante Druck, den der Cuff auf die Tracheawand ausübt, zu Veränderungen oder Verletzungen der Tracheawand führen kann, wird in der Regel nur dann eine Trachealkanüle mit Cuff verwendet, wenn dies auch medizinisch induziert ist, also beispielsweise bei Schluckstörungen (Speichel/Nahrung könnte in die Lunge gelangen) oder anderweitiger Sekretbildung. Es ist daher denkbar, dass die Trachealkanüle mit oder ohne Cuff ausgeführt ist.

Nach einer vorteilhaften Ausführungsform ist der Cuff an der Innenwandung des Hohlkörperelements angeordnet. Die Außenwandung schließt vorteilhafterweise an der Oberfläche des Cuffs ab. Nach einer weiteren Ausführungsform ist der Cuff an der Außenwandung des Hohlkörperelements angeordnet. Denkbar wäre auch, dass der Cuff an der Innenwandung und an der Außenwandung des Hohlkörperelements angeordnet ist.

Nach einer weiteren vorteilhaften Ausführungsform ist der Cuff mit zumindest einem Stabilisierungskanal fluidisch verbunden. Durch eine solche fluidische Verbindung kann vorzugsweise die Expansion des Cuffs kontrolliert werden. Das Expansionsfluid würde dann dem Stabilisierungsfluid entsprechen. Alternativ oder kumulativ ist eine zweite Zugangseinrichtung vorgesehen, mittels welcher die Expansion des Cuffs kontrollierbar ist. Die zweite Zugangseinrichtung umfasst vorzugsweise ein Ventileinrichtung mittels welcher die Menge an Expansionsfluid in dem Cuff variierbar ist. Ferner kann vorteilhafterweise der Zugang zu dem Cuff durch diese Ventileinrichtung verschlossen werden.

Nach einer weiteren vorteilhaften Ausführungsform weisen die Innenwandung und/oder die Außenwandung zumindest einen Verstärkungsabschnitt auf. Ein solcher Verstärkungsabschnitt kann durch ein Einbringen eines weiteren Materials auf oder in die Innenwandung und/oder die Außenwandung erfolgen. Dieses weitere Material hat formstabile oder starre Eigenschaften und/oder weist eine geringere Elastizität als die in der restlichen Wandung verwendeten Materialeien auf. Vorzugsweise weisen die Innenwandung und/oder die Außenwandung einen Verstärkungsabschnitt im Bereich des anliegenden Cuffs auf. Da in dem Bereich der Innenwandung und/oder die Außenwandung, an welchem vorteilhafterweise der Cuff angeordnet ist, durch den Cuff ein erhöhter Krafteintrag in das Hohlkörperelement erfolgen kann, kann der Verstärkungsabschnitt die Innenwandung und/oder die Außenwandung entsprechend stabilisieren.

Bei Trachealkanülen kommt es nach einer gewissen Nutzungsdauer leicht zu Sekretansammlungen im Strömungspfad der Kanüle. Dies stellt für den beatmeten Patienten eine große Gefahr dar, da dieser in diesem Fall nur schlecht oder sogar gar nicht mehr beatmet werden kann und die Gefahr des Erstickens besteht. Besonders bei Trachealkanülen mit aufgeblasenen Cuff stellt der Strömungspfad der Trachealkanüle den einzigen Luftweg in die Lunge des Patienten hinein oder aus dieser heraus dar. Zwar ist es möglich den Cuff in diesem Fall zu entblocken, also die Luft aus dem Cuff abzulassen, sodass der Patient prinzipiell über die oberen Atemwege beatmet werden kann. Dies ist jedoch nur möglich, wenn die oberen Atemwege des Patienten frei sind bzw. der Patient über die oberen Atemwege beatembar ist, was bei einem Patienten mit Tracheostoma aber in der Regel nicht der Fall ist.

Bisher werden häufig Trachealkanülen mit austauschbaren Innenseelen verwendet. Im Falle einer Verstopfung des Strömungspfads wird die verstopfte Innenseele gegen eine neue, freie Innenseele ausgetauscht. Diese Vorgehensweise weist allerdings mehrere Nachteile auf. Zum einen muss immer eine neue Innenseele für den notfallmäßigen Austausch bereit liegen. Bei intensivmedizinisch betreuten Patienten mag dies noch problemlos möglich sein. Bei Patienten, die zu Hause beatmet werden und ggf. selbst oder mit Hilfe eines Rollstuhls mobil sind, gestaltet sich das ständige Bereithalten einer neuen Innenseele bereits schwieriger. Auch können Patienten, Angehörige oder nicht intensivmedizinisch geschultes Pflegepersonal unter Umständen die Innenseele nicht selbst tauschen oder trauen sich dies im Notfall nicht zu. Das Tauschen der Innenseele sollte außerdem steril erfolgen, was in einer Notfallsituation häufig nicht möglich ist. Auch muss der Patient für den Tausch der Innenseele vom Beatmungsgerät getrennt werden, was ebenfalls eine zusätzliche Gefahr für den Patienten darstellt. Zuletzt geht die Verwendung von Kanülen mit Innenseele bei gleichem Innendurchmesser der Trachealkanüle immer mit einer Erhöhung des Außendurchmessers der Kanüle einher, sodass diese Kanülen auf Grund der Anatomie nicht bei jedem Patienten anwendbar sind.

Nach einer weiteren bevorzugten Ausführungsform ist an einer inneren Oberfläche der Innenwandung eine Verschlussschicht angeordnet. Vorzugsweise ist zwischen der inneren Oberfläche der Innenwandung und der Verschlussschicht ein zweites Zwischenlumen ausgebildet. Bevorzugt ist das zweite Zwischenlumen über eine dritte Zugangseirichtung zugänglich. Vorteilhafterweise ist in einem Grundzustand ein Unterdruck in dem Zwischenlumen einstellbar. Durch das vorteilhafte Einstellen des Unterdrucks liegt die Verschlussschicht glatt an der Innenwandung an und beeinträchtigt demnach den Strömungspfad des Atemgases nicht. Die Verschlussschicht kann aus einem handelsüblichen Material, welches zur Herstellung von Trachealkanülen verwendet wird, bestehen. Insbesondere kann die Verschlussschicht aus dem gleichen Material wie die Innenwandung und/oder die Außenwandung bestehen.

Dabei ist es von Vorteil, dass in einem Verschlusszustand ein zweites Expansionsfluid in das zweite Zwischenlumen einbringbar ist. Bevorzugt ist das zweite Expansionsfluid mittels der dritten Zugangseirichtung einbringbar. Durch die Erhöhung der Menge an zweiten Expansionsfluid expandiert das Zwischenlumen entsprechend. Die Verschlussschicht wird durch die Expansion radial nach innen bewegt. Die inneren Oberflächen der Verschlussschicht liegen demnach vorteilhafterweise aneinander an. Durch die Expansion des Zwischenlumens ist das Innenlumen des Hohlkörperelements verkleinerbar oder gar verschließbar. Der Verschluss kann temporär annähernd vollständig sein.

Durch das Verkleinern, beziehungsweise Verschließen des Innenlumens mittels der Verschlussschicht wird ein Schleimpfropf, der sich im Hohlkörperelement befindet, gequetscht und insbesondere in die Länge gezogen. Nach dem anschließenden Öffnen des Verschlusses durch die Verschlussschicht nimmt der gequetschte Schleimpfropf nicht mehr den gesamten Innenquerschnitt des Hohlkörperelements ein, sodass wieder ein Strömungspfad für die Beatmung zur Verfügung steht. Es ist somit im Falle einer Verstopfung der Trachealkanüle nicht notwendig die Kanüle oder die Innenseele zu entfernen bzw. zu ersetzen. Es wird somit eine vorteilhafte Trachealkanüle bereitgestellt, die im Fall einer Verstopfung auf einfache Art und Weise entblockbar ist. Dies ist selbst von ungeschultem Pflegepersonal oder sogar vom Patienten selbst sicher, einfach und schnell durchführbar ist, ohne den Patienten vom Beatmungsgerät trennen zu müssen.

Die dritte Zugangseinrichtung umfasst vorzugsweise ein Ventileinrichtung mittels welcher die Menge an Expansionsfluid in dem zweiten Zwischenlumen variierbar ist. Ferner kann durch die Ventileinrichtung ein Unterdruck in dem zweiten Zwischenlumen erzeugt werden. Dies kann beispielsweise durch ein Verbinden der Ventileinrichtung mit einer Spritze oder ähnlichem erfolgen.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig. 1: eine perspektivische Ansicht einer Beatmungsvorrichtung nach einer Ausführungsform;
- Fig. 2: eine Seitenansicht einer Beatmungsvorrichtung nach einer Ausführungsform;
- Fig. 3: eine Seitenansicht einer Beatmungsvorrichtung nach einer Ausführungsform;
- Fig. 4: eine Frontansicht einer Beatmungsvorrichtung nach einer Ausführungsform;
- Fig. 5: eine perspektivische Ansicht einer Beatmungsvorrichtung nach einer Ausführungsform;
- Fig. 6: eine Seitenansicht einer Beatmungsvorrichtung nach einer Ausführungsform;
- Fig. 7: eine Frontansicht einer Beatmungsvorrichtung nach einer Ausführungsform;
- Fig. 8: eine Schnittansicht einer Beatmungsvorrichtung nach einer Ausführungsform;
- Fig. 9: eine Schnittansicht einer Beatmungsvorrichtung nach einer Ausführungsform;
- Fig. 10: eine Schnittansicht des Hohlkörperelements nach einer Ausführungsform;
- Fig. 11: eine Schnittansicht des Hohlkörperelements nach einer Ausführungsform;
- Fig. 12: eine Schnittansicht des Hohlkörperelements nach einer Ausführungsform;
- Fig. 13: eine Schnittansicht des Hohlkörperelements nach einer Ausführungsform;
- Fig. 14: eine Schnittansicht des Hohlkörperelements nach einer Ausführungsform;
- Fig. 15: eine Schnittansicht des Hohlkörperelements nach einer Ausführungsform;
- Fig. 16: eine Schnittansicht des Hohlkörperelements nach einer Ausführungsform;

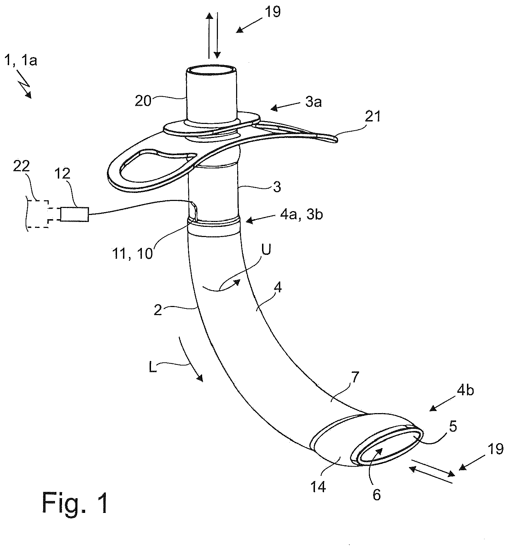

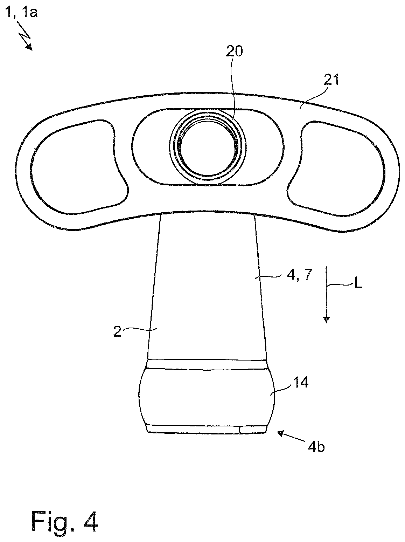

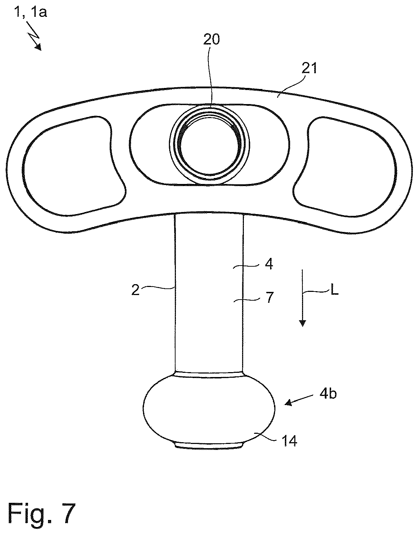

In den Figuren 1 bis 16 ist Beatmungsvorrichtung 1, insbesondere eine Trachealkanüle 1a zum Einsetzen in ein Tracheostoma dargestellt, umfassend ein Kanülenelement 2, welches ein Halselement 3 und ein an dem Halselement 3 angeordnetes Hohlkörperelement 4 umfasst, wobei das Hohlkörperelement 4 eine Innenwandung 5 aufweist, welche zumindest ein Innenlumen 6 umgibt, wobei die Innenwandung 5 von einer Außenwandung 7 umgeben ist, wobei zwischen der Innenwandung 5 und der Außenwandung 7 ein Zwischenlumen 8 definiert ist, in welchem zumindest ein rohrartig ausgebildeter Stabilisierungskanal 9 angeordnet ist, wobei in einem ersten Zustand das Hohlkörperelement 4 in einem flexiblen Zustand ist, wobei durch Zuführen einer vorbestimmten Menge an Stabilisierungsfluid in die Stabilisierungskanäle 9 das Hohlkörperelement 4 in einen zweiten formstabilen Zustand bringbar ist.

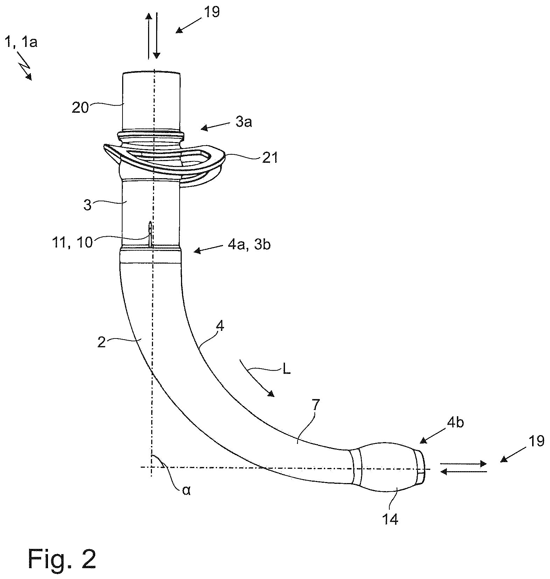

Das Halselement 3 umfasst ein distales Ende 3a und ein proximales Ende 3b. An dem proximalen Ende 3b des Halselement 3 ist dabei ein distales Ende 4a des Hohlkörperelements 4 angeordnet. Das Kanülenelement 2, beziehungsweise das Halselement 3 und das Hohlkörperelement 4 umschließen einen fluidischen Strömungspfad, welcher sich von dem distalen Ende des Kanülenelements 2, beziehungsweise von dem distalen Ende 3a des Halselements 3 zu dem proximalen Ende des Kanülenelements 2, beziehungsweise dem proximalen Ende 4b des Hohlkörperelements 4 erstreckt. In den Figuren 1, 2 und ist ein Doppelpfeil 19 dargestellt, welcher den Eintritt und den Austritt des Fluids in den Strömungspfad darstellen soll. Die Strömung kann dabei in beiden Richtungen stattfinden. Vorzugsweise ist dieses Fluid Luft oder Atemluft.

An dem distalen Ende 3a des Halselements 3 sind ein Kanülenkonnektor 20 und ein Kanülenschild 21 angeordnet. Ein solcher Kanülenschild 21 dient zur Befestigung der Kanüle 1a am Hals des Patienten. An dem Kanülenkonnektor 20 kann beispielsweise ein Beatmungsschlauch oder ein Aufsatz in Form eines Wärmefeuchtigkeitsaustauscher angebracht sein.

Das Hohlkörperelement 4, beziehungsweise das Kanülenelement 2 weist in dem zweiten Zustand einen gekrümmten Verlauf auf. Dabei weist das Hohlkörperelement 4 einen Krümmungswinkel α auf welcher in einem Bereich von 90°-120° liegt. Der Krümmungswinkel ist explizit in Figur 2 dargestellt. Der Krümmungswinkel α erstreckt sich demnach zwischen einer entsprechenden Mittelachse eines distalen und eines proximalen Abschnitts. In der Regel wird der Krümmungswinkel vom individuellen Verlauf der Luftröhre abhängig gemacht. Bei Erwachsenen wird jedoch meist ein Krümmungswinkel von 90° gewählt, bei Kindern einer im Bereich von 105°-120°. Vorzugsweise passt sich das Kanülenelement 2, beziehungsweise das Hohlkörperelement 4, beziehungsweise der Krümmungswinkel dieser während des Einführens des Stabilisierungsfluids in die Stabilisierungskanäle 9 an den Verlauf der Trachea an.

Das Hohlkörperelement 4 ist ähnlich einer Doppelwandkonstruktion ausgebildet, wobei in dem ersten Zwischenlumen vorzugsweise eine Mehrzahl an Stabilisierungskanälen 9 angeordnet ist. In dem ersten Zustand ist das Hohlkörperelement in einem flexiblen ersten Zustand (nicht dargestellt in den Figuren). In einem zweiten Zustand ist das Hohlkörperelement in einen formstabilen, beziehungsweise starren Zustand. Durch ein Einfließen des Stabilisierungsfluids stabilisiert sich das Hohlkörperelement in Baurichtung.

Das Stabilisierungsfluid ist über zumindest eine erste Zugangseinrichtung 10 dem zumindest einem Stabilisierungskanal 9 zuführbar und/oder aus dem zumindest einem Stabilisierungskanal 9 abführbar. An dem distalen Ende 4a des Hohlkörperelements 4 besteht eine fluidische Verbindung zwischen der zumindest einen Zugangseinrichtung 10 und zumindest einer Teilmenge an Stabilisierungskanälen 9. Üblicherweise ist das Stabilisierungsfluid Luft.

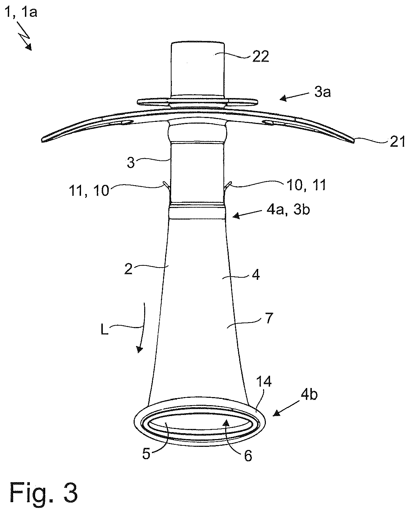

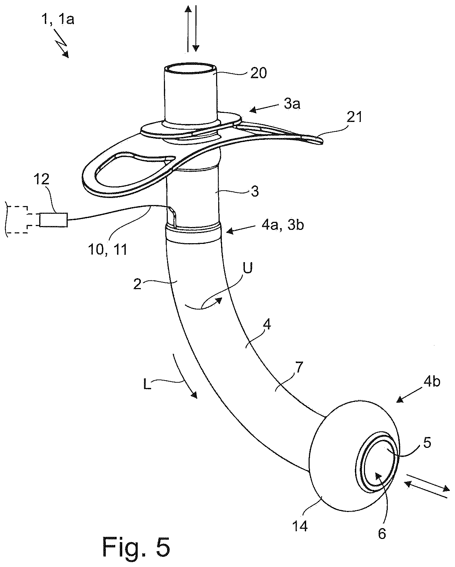

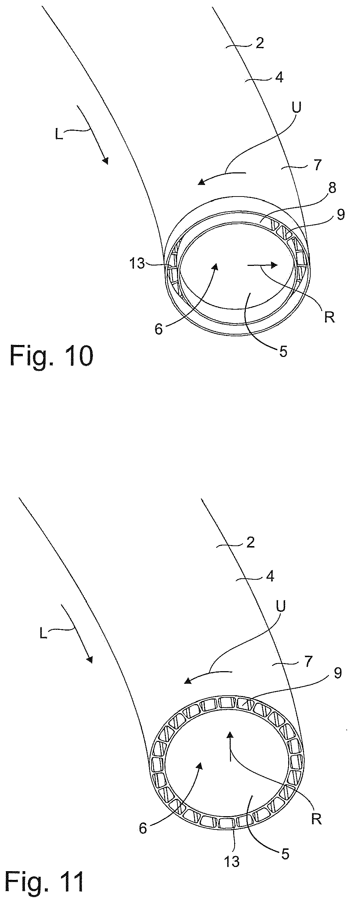

Die erste Zugangseinrichtung 10 umfasst zumindest eine aus der Beatmungsvorrichtung 1 führende Verbindungseinrichtung 11. Diese Verbindungseinrichtung 11 tritt durch eine Außenwandung des Halselements 3. In den Ausführungsformen gemäß den Figuren 3 und 11 sind zwei diametral gegenüber liegende Verbindungseinrichtungen 11 vorgesehen. Diese können jeweils zu einer Zugangseinrichtung 10 gehören oder zu unterschiedlichen Zugangseinrichtungen. In dem erst genannten Fall sind die Verbindungseinrichtung 11, welche üblicherweise als Schläuche oder Röhrchen ausgebildet sein können, mit einer Ventileinrichtung 12 verbunden. Mittels dieser Ventileinrichtung 12 ist der Zugang verschließbar und der Innendruck, beziehungsweise die Menge an Stabilisierungsfluid in den Stabilisierungskanälen variierbar. Denkbar wäre, dass die Ventileinrichtung 12 mit einer üblichen Spritze 24a, angedeutet in den Figuren 1 und 5 verbindbar ist. Die Modifikation der Menge an Stabilisierungsfluid kann dann mit Hilfe der Spritze 24a erfolgen.

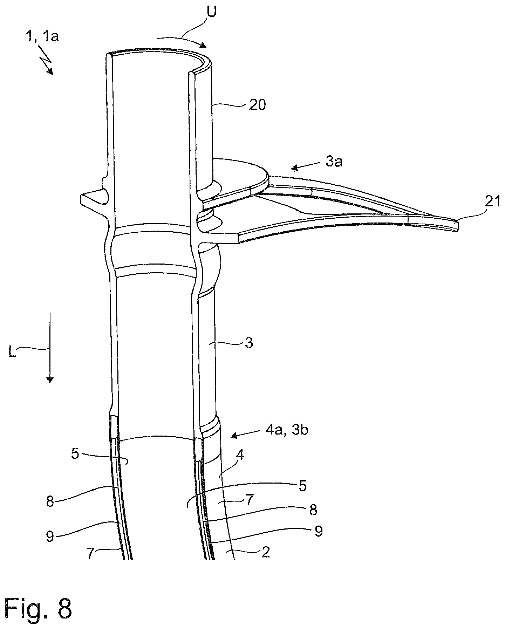

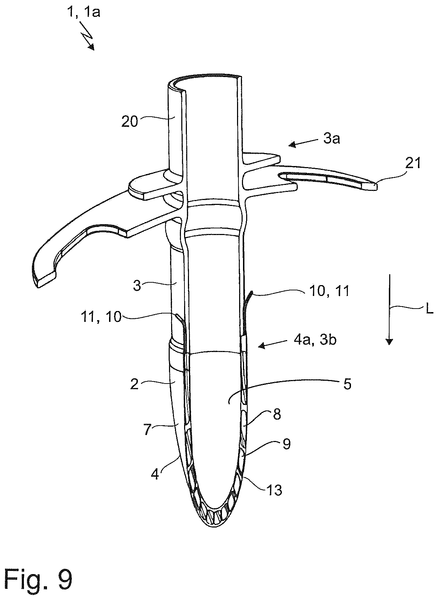

Der zumindest eine Stabilisierungskanal, beziehungsweise die Stabilisierungskanäle 9 können auf verschiedenartige Weise orientiert, beziehungsweise ausgebildet sein. Ferner können mehrere Teilmengen an Stabilisierungskanälen 9 vorgesehen sein, welche eine unterschiedliche Orientierung relativ zu dem Hohlkörperelement 4 aufweisen und/oder unterschiedlich ausgebildet sind. Nach einer Ausführungsform erstreckt sich zumindest eine Teilmenge von Stabilisierungskanäle 9 im Wesentlichen ausgehend von dem distalen Ende 4a des Hohlkörperelements 4. Diese Stabilisierungskanäle 9 münden dann im Wesentlichen am proximalen Ende 4b des Hohlkörperelements 4. Denkbar wäre auch, dass mehrere Teilmengen an Stabilisierungskanälen 9 vorgesehen sind, wobei zumindest eine zweite Teilmenge nicht von dem distalen Ende 4a des Hohlkörperelements 4 ausgehend ist. Hier ist es vorteilhaft, wenn eine fluidische Verbindung zwischen der zweiten Teilmenge an Stabilisierungskanälen 9 und der zumindest einen ersten Teilmenge an Stabilisierungskanälen 9, welche mit der ersten Zugangseinrichtung 10 am distalen Ende 4a des Hohlkörperelements 4 verbunden ist, vorgesehen ist. Somit wird eine entsprechende Strömung des Stabilisierungsfluids in die zweite Teilmenge an Stabilisierungskanälen 9 gewährleistet. Vorteilhafterweise erfolgt eine Strömung von Stabilisierungsfluid durch die Stabilisierungskanäle 9 von dem distalen Ende 4a zu dem proximalen Ende 4b des Hohlkörperelements 4. Die Strömung kann dabei, wie schon erwähnt, auch durch fluidische Verbindungen zwischen den Stabilisierungskanälen 9 erfolgen. Somit wird eine gleichmäßige Verteilung, beziehungsweise Befüllung des Stabilisierungsfluids in den Stabilisierungskanälen erreicht. Zumindest eine Teilmenge der Stabilisierungskanäle 9 kann sich im Wesentlichen entlang einer Längsrichtung L des Hohlkörperelements 4 erstrecken. Zumindest eine Teilmenge der Stabilisierungskanäle 9 kann sich im Wesentlichen geradlinig oder helixartig entlang der Längsrichtung L des Hohlkörperelements 4 erstrecken. Dabei mündet zumindest eine Teilmenge der Stabilisierungskanäle 9 in einem proximalen Verschluss. In der Figur 8 ist eine Schnittdarstellung der Beatmungsvorrichtung 1 dargestellt. Hier verlaufen alle Stabilisierungskanäle 9 entlang der Längsrichtung L ausgehend im Wesentlichen von dem distalen Ende 4a des Hohlkörperelements bis zu im Wesentlichen dem proximalen Ende 4b des Hohlkörperelements 4. Die Stabilisierungskanäle 9 können dabei entlang einer Umfangsrichtung U nebeneinanderliegend angeordnet sein. Dies ist beispielsweise in Figur 11 dargestellt. In Figur 9 ist eine Ausführungsform dargestellt, in welcher zumindest ein Stabilisierungskanal 9 sich helixartig entlang der Längsrichtung L erstreckt. Der zumindest eine Stabilisierungskanal erstreckt sich ausgehend von dem distalen Ende 4a des Hohlkörperelements zu dem proximalen Ende 4b des Hohlkörperelements 4.

Der zumindest eine Stabilisierungskanal 9 kann durch zumindest ein Stegelement 13, bevorzugt zwei Stegelemente 13 und einem Abschnitt der Innenwandung 5 und einem Abschnitt der Außenwandung 7 gebildet werden. Das zumindest eine Stegelement 13 ist zwischen der Innenwandung 5 und der Außenwandung 7 angeordnet. Das zumindest eine Stegelemente 13 erstreckt sich dabei im Wesentlichen entlang einer radialen Richtung R. In den Figuren 9, 10 und 11 sind die Stegelemente 13 ersichtlich.

In der Figur 10 ist eine Ausführungsform dargestellt, welche Stabilisierungskanäle 9 mit unterschiedlichen Ausmaßen entlang der Umfangsrichtung aufweist. Die Ausmaße, die Querschnitte, beziehungsweise die Form der Querschnitte, können beliebig an die jeweiligen Anforderungen der Trachealkanüle angepasst werden.

Alternativ oder kumulativ kann der zumindest eine Stabilisierungskanal zwischen der Innenwandung 5 und der Außenwandung 7 vorgesehenes Röhrenelement gebildet werden.

Zwischen der Innenwandung 5 und der Außenwandung 7 ist zumindest eine Zwischenschicht vorgesehen, die freilegend zwischen der Innenwandung 5 und der Außenwandung angeordnet sein oder mit der Innenwandung 5 und/oder der Außenwandung verbunden sein kann.

An einem proximalen Ende 4b des Hohlkörperelements 4 ist ein Cuff 14 angeordnet. Der Cuff 14 ist zu einer vorbestimmten Größe expandierbar. In den Figuren 1 bis 4 ist dabei ein nicht expandierter Cuff (14) dargestellt und in den Figuren 5 bis 7 ein expandierter Cuff 14. Die Expansion kann durch Zuführung eines Expansionsfluids erfolgen. Hierzu kann der Cuff 14 mit zumindest einem Stabilisierungskanal 9 fluidisch verbunden sein. Die Kontrolle der Expansion des Cuffs 14 kann über den zumindest einem Stabilisierungskanal 9 erfolgen. Alternativ oder kumulativ kann eine zweite Zugangseinrichtung 15 vorgesehen sein, mittels welcher die Expansion des Cuffs kontrollierbar ist.

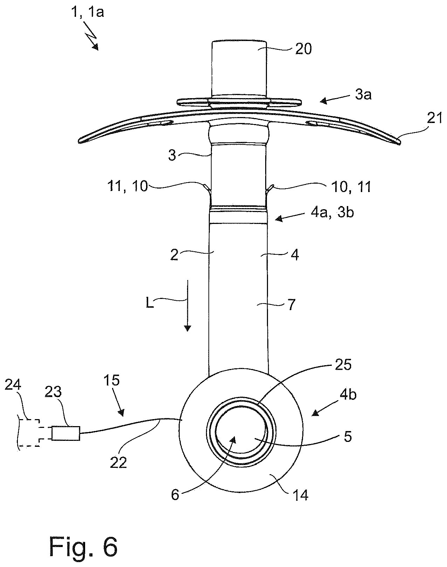

Die zweite Zugangseinrichtung 15 weist zumindest eine aus der Beatmungsvorrichtung 1 führende zweite Verbindungseinrichtung 22 auf. Diese Verbindungseinrichtung 22 kann als Schlauch oder als ein Röhrchen ausgebildet sein. Dies ist beispielsweise in Figur 6 exemplarisch dargestellt. Die Verbindungseinrichtung 22 ist mit einer zweiten Ventileinrichtung 23 verbunden. Mittels dieser Ventileinrichtung 23 ist der Zugang verschließbar und der Innendruck, beziehungsweise die Menge an Expansionsfluid, in dem Cuff 14 variierbar. Denkbar wäre, dass die Ventileinrichtung 23 mit einer üblichen Spritze 24b verbindbar ist. Die Modifikation der Menge an Expansionsfluid kann dann mit Hilfe der Spritze 24b erfolgen.

Der Cuff 14 kann an der Innenwandung 5 und/oder der Außenwandung 7 angeordnet sein. Vorzugsweise ist der Cuff 14 mittels einer stoffschlüssigen Verbindung an der Innenwandung 5 und/oder der Außenwandung 7 angeordnet.

Die Innenwandung 5 und/oder die Außenwandung 7 zumindest kann einen Verstärkungsabschnitt 25 aufweisen. Insbesondere können die Innenwandung 5 und/oder die Außenwandung 7 einen Verstärkungsabschnitt 25 im Bereich des anliegenden Cuffs 14 aufweisen. Durch einen solchen Verstärkungsabschnitt 25 können die Kräfte, welche durch den Cuff 14 auf das Hohlkörperelement eingebracht werden, berücksichtigt werden, wodurch eine Stabilität des Hohlkörperelements 4 gewährleistet wird.

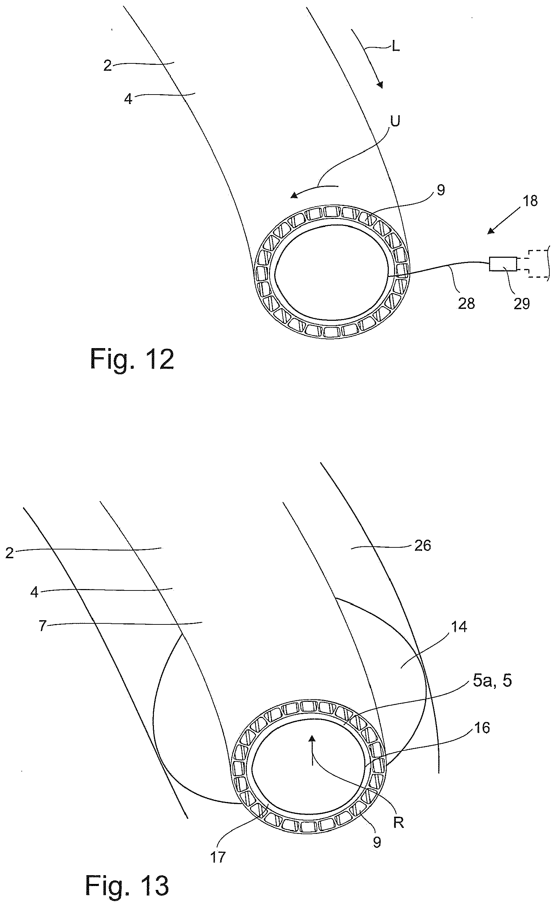

In den Figuren 12 bis 16 ist eine Ausführungsform dargestellt, mittels welcher auf einfache Art und Weise eine Verstopfung des Innenlumens 6, beispielsweise durch einen Schleimpfropf entblockt werden kann. Dies ist selbst von ungeschultem Pflegepersonal oder sogar vom Patienten selbst sicher, einfach und schnell durchführbar, ohne den Patienten vom Beatmungsgerät trennen zu müssen. Dargestellt ist auch prinzipiell die Trachea 26, in welcher die Beatmungsvorrichtung 1 angeordnet ist.

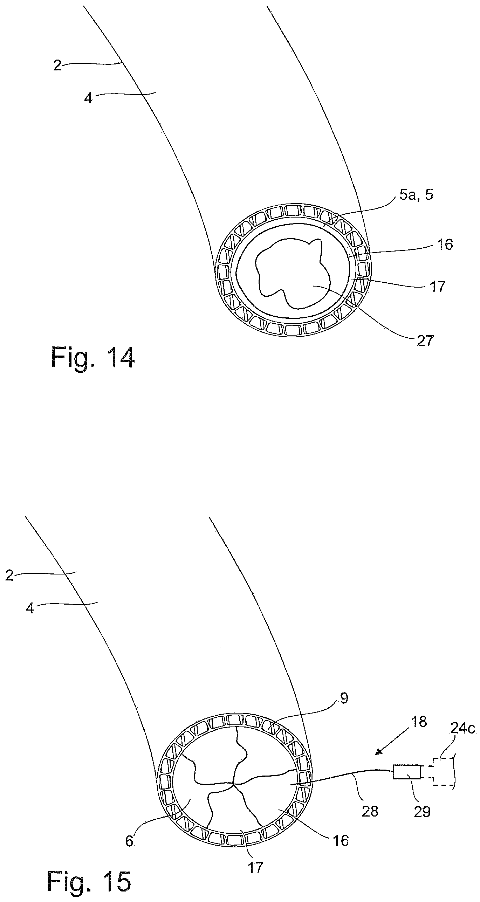

Hierzu ist an einer inneren Oberfläche 5a der Innenwandung 5 eine Verschlussschicht 16 angeordnet. Zwischen der inneren Oberfläche 5a der Innenwandung 5 und der Verschlussschicht 16 ist ein zweites Zwischenlumen 17 ausgebildet. Das Zwischenlumen 17 ist über eine dritte Zugangseirichtung 18 zugänglich. In einem Grundzustand ist ein Unterdruck in dem zweiten Zwischenlumen 17 einstellbar, wodurch die Verschlussschicht 16 eng an der inneren Oberfläche 5a anliegt. Hierdurch wird die Strömung des Atemgases nicht durch die Verschlussschicht 16 beeinträchtigt. Zwischen der inneren Oberfläche 5a und der Verschlussschicht 16 sind bevorzugt Befestigungs-, beziehungsweise Abschlussverbindungen vorgesehen, damit das zweite Zwischenlumen 17 abgeschlossen werden kann. In Figur 14 ist ein Hohlkörperelement 4 gezeigt, in welchem eine Verstopfung 27, beispielsweise ein Schleimpfropf oder ein Sekretpfropf vorhanden ist. In einem Verschlusszustand ist ein zweites Expansionsfluid in das zweite Zwischenlumen 17 einbringbar, wodurch dieses expandiert Durch die Expansion des Zwischenlumens 17 ist das Innenlumen 6 des Hohlkörperelements 4 verkleinerbar, beziehungsweise verschließbar.

Die dritte Zugangseinrichtung 18 weist zumindest eine aus der Beatmungsvorrichtung 1 führende dritte Verbindungseinrichtung 28 auf. Diese Verbindungseinrichtung 28 kann als Schlauch oder als ein Röhrchen ausgebildet sein. Dies ist beispielsweise in den Figuren 12 und 15 exemplarisch dargestellt. Die Verbindungseinrichtung 28 ist mit einer dritten Ventileinrichtung 29 verbunden. Mittels dieser Ventileinrichtung 28 ist der Zugang verschließbar und der Innendruck, beziehungsweise die Menge an Expansionsfluidsfluid in den in dem zweiten Zwischenlumen 17 variierbar. Denkbar wäre, dass die Ventileinrichtung 28 mit einer üblichen Spritze 24c verbindbar ist. Die Modifikation der Menge an Expansionsfluid kann dann mit Hilfe der Spritze 24c erfolgen.

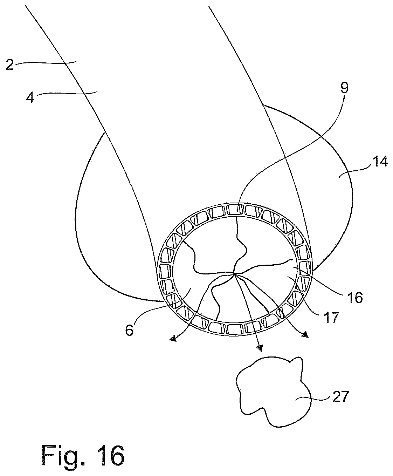

In den Figuren 15 und 16 wird der Verschlusszustand dargestellt. Durch die Expansion des Zwischenlumens 17 verkleinert, beziehungsweise verschließt die Verschlussschicht 16 das Innenlumen 6. Durch das Verkleinern, beziehungsweise Verschließen des Innenlumens 6 mittels der Verschlussschicht 16, wird die Verstopfung 27, der sich im Hohlkörperelement 4 befindet gequetscht und insbesondere in die Länge gezogen. Nach dem anschließenden Öffnen des Verschlusses durch die Verschlussschicht 16 nimmt der gequetschte Schleimpfropf nicht mehr den gesamten Innenquerschnitt des Hohlkörperelements 4 ein, sodass wieder ein Strömungspfad für die Beatmung zur Verfügung steht. Durch das Verkleinern des Innenvolumens kann die Verstopfung 27 aus dem Innenlumen 6 gedrängt werden.

### Bezugszeichenliste

- 1: Beatmungsvorrichtung
- 1a: Trachealkanüle
- 2: Kanülenelement
- 3: Halselement
- 3a: distales Ende des Halselements
- 3b: proximales Ende des Halselements
- 4: Hohlkörperelement
- 4a: distales Ende des Hohlkörperelements
- 4b: proximales Ende des Hohlkörperelements
- 5: Innenwandung
- 5a: innere Oberfläche der Innenwandung
- 6: Innenlumen
- 7: Außenwandung
- 8: Zwischenlumen
- 9: Stabilisierungskanäle
- 10: erste Zugangseinrichtung
- 11: Verbindungseinrichtung
- 12: Ventileinrichtung
- 13: Stegelemente
- 14: Cuff
- 15: zweite Zugangseinrichtung
- 16: Verschlussschicht
- 17: zweites Zwischenlumen
- 18: dritte Zugangseirichtung
- 19: Doppelfeil
- 20: Kanülenkonnektor
- 21: Kanülenschild
- 22: zweite Verbindungseinrichtung
- 23: dritte Ventileinrichtung
- 24a: Spritze
- 24b: Spritze
- 24c: Spritze
- 25: Verstärkungsabschnitt
- 26: Trachea
- 27: Verstopfung
- 28: dritte Verbindungseinrichtung
- 29: dritte Ventileinrichtung
- α: Neigungswinkel
- L: Längsrichtung
- U: Umfangsrichtung
- R: radiale Richtung

## Patentansprüche

1. Beatmungsvorrichtung (1), insbesondere eine Trachealkanüle (1a) zum Einsetzen in ein Tracheostoma, umfassend ein Kanülenelement (2), welches ein Halselement (3) und ein an dem Halselement (3) angeordnetes Hohlkörperelement (4) umfasst, wobei das Hohlkörperelement (4) eine Innenwandung (5) aufweist, welche zumindest ein Innenlumen (6) umgibt,
wobei
die Innenwandung (5) von einer Außenwandung (7) umgeben ist, wobei zwischen der Innenwandung (5) und der Außenwandung (7) ein Zwischenlumen (8) definiert ist, wobei in einem ersten Zustand das Hohlkörperelement (4) in einem flexiblen Zustand ist, wobei durch Zuführen einer vorbestimmten Menge an Stabilisierungsfluid in das Zwischenlumen (8) das Hohlkörperelement (4) in einen zweiten formstabilen Zustand bringbar ist, wobei zwischen der Innenwandung (5) und der Außenwandung (7) zumindest eine Zwischenschicht vorgesehen ist.

2. Beatmungsvorrichtung (1) nach Anspruch 1,
wobei
in dem Zwischenlumen (8) zumindest ein Stabilisierungskanal (9) angeordnet ist, wobei durch Zuführen einer vorbestimmten Menge an Stabilisierungsfluid in den zumindest einem Stabilisierungskanal (9) das Hohlkörperelement (4) in einen zweiten formstabilen Zustand bringbar ist, wobei das Stabilisierungsfluid über zumindest eine Zugangseinrichtung (10) dem zumindest einem Stabilisierungskanal (9) zuführbar und/oder aus dem zumindest einem Stabilisierungskanal (9) abführbar ist, wobei an einem distalen Ende (4a) des Hohlkörperelements (4) eine fluidische Verbindung zwischen der zumindest einen Zugangseinrichtung (10) und dem zumindest einem Stabilisierungskanal (9) besteht, wobei das Stabilisierungsfluid Luft ist.

3. Beatmungsvorrichtung (1) nach Anspruch 2,
wobei
die zumindest eine Zugangseinrichtung (10), zumindest eine aus der Beatmungsvorrichtung (1) führende Verbindungseinrichtung (11) umfasst, wobei die zumindest eine Verbindungseinrichtung (11) durch eine Außenwandung des Halselements (3) tritt, wobei die zumindest eine Zugangseinrichtung eine Ventileinrichtung (12) umfasst.

4. Beatmungsvorrichtung (1) nach einem der Ansprüche 2 bis 3
wobei
der zumindest eine Stabilisierungskanal (9) eine Erstreckung aufweist, welche im Wesentlichen von einem distalen Ende (4a) des Hohlkörperelements (4) ausgeht, wobei der zumindest eine Stabilisierungskanal (9) sich im Wesentlichen bis zu einem proximalen Ende (4b) des Hohlkörperelements (4) erstreckt, wobei der zumindest eine Stabilisierungskanal (9) sich im Wesentlichen entlang einer Längsrichtung (L) des Hohlkörperelements (4) erstreckt, wobei der zumindest eine Stabilisierungskanal (9) sich im Wesentlichen geradlinig oder helixartig entlang der Längsrichtung (L) des Hohlkörperelements (4) erstreckt, wobei der zumindest eine Stabilisierungskanal (9) in einem proximalen Verschluss mündet.

5. Beatmungsvorrichtung (1) nach einem der Ansprüche 2 bis 4,
wobei
zwischen der Innenwandung (5) und der Außenwandung (7) zumindest ein Stegelement (13) angeordnet ist, wobei das zumindest eine Stegelement (13) sich im Wesentlichen entlang einer radialen Richtung (R) des Hohlkörperelements (4) erstreckt, wobei der zumindest eine Stabilisierungskanal (9) durch einen Abschnitt der Innenwandung (5), einen Abschnitt der Außenwandung (7) und dem zumindest einem Stegelement (13) gebildet ist.

6. Beatmungsvorrichtung (1) nach einem der Ansprüche 2 bis 5,
wobei
zwischen der Innenwandung (5) und der Außenwandung (7) zumindest ein Röhrenelement vorgesehen ist, welches den zumindest einen Stabilisierungskanal (9) bildet.

7. Beatmungsvorrichtung (1) nach einem der vorhergehenden Ansprüche,
wobei
die zumindest eine Zwischenschicht freilegend zwischen der Innenwandung (5) und/oder der Außenwandung (7) ist.

8. Beatmungsvorrichtung (1) nach einem der vorhergehenden Ansprüche,
wobei
an einem proximalen Ende (4b) des Hohlkörperelements (4) ein Cuff (14) angeordnet ist, wobei der Cuff (14) zu einer vorbestimmten Größe expandierbar ist, wobei die Expansion durch Zuführung eines Expansionsfluids erfolgt, wobei der Cuff (14) an der Innenwandung (5) und/oder der Außenwandung (7) angeordnet ist.

9. Beatmungsvorrichtung (1) nach Anspruch 8,
wobei
der Cuff (14) mit zumindest einem Stabilisierungskanal (9) fluidisch verbunden ist, wobei eine Kontrolle der Expansion des Cuffs (14) über den zumindest einem Stabilisierungskanal (9) erfolgen kann, wobei eine zweite Zugangseinrichtung (15) vorgesehen ist mittels welcher die Expansion des Cuffs (14) kontrollierbar ist.

10. Beatmungsvorrichtung (1) nach einem der vorhergehenden Ansprüche
wobei
die Innenwandung (5) und/oder die Außenwandung (7) zumindest einen Verstärkungsabschnitt (25) aufweisen, wobei die Innenwandung (5) und/oder die Außenwandung (7) einen Verstärkungsabschnitt (25) im Bereich des anliegenden Cuffs (14) aufweist.

11. Beatmungsvorrichtung (1) nach einem der vorhergehenden Ansprüche
wobei
an einer inneren Oberfläche (5a) der Innenwandung (5) eine Verschlussschicht (16) angeordnet ist, wobei zwischen der inneren Oberfläche (5a) der Innenwandung (5) und der Verschlussschicht (16) ein zweites Zwischenlumen (17) ausgebildet ist, wobei das zweite Zwischenlumen (17) über eine dritte Zugangseirichtung (18) zugänglich ist, wobei in einem Grundzustand ein Unterdruck in dem zweiten Zwischenlumen (17) einstellbar ist

12. Beatmungsvorrichtung (1) nach Anspruch 11,
wobei
in einem Verschlusszustand ein zweites Expansionsfluid in das zweite Zwischenlumen (17) einbringbar ist, wodurch dieses expandiert, wobei durch die Expansion des Zwischenlumens das Innenlumen (6) des Hohlkörperelements (4) verkleinerbar ist.

## Claims

1. A ventilation device (1), in particular a tracheal cannula (1a) for insertion into a tracheostoma, comprising a cannula element (2) which comprises a neck element (3) and a hollow body element (4) arranged on the neck element (3), wherein the hollow body element (4) has an inner wall (5) which surrounds at least one inner lumen (6),
wherein
the inner wall (5) is surrounded by an outer wall (7), wherein an intermediate lumen (8) is defined between the inner wall (5) and the outer wall (7), wherein in a first state the hollow body element (4) is in a flexible state, wherein the hollow body element (4) can be brought into a second dimensionally stable state by supplying a predetermined amount of stabilising fluid into the intermediate lumen (8), wherein at least one intermediate layer is provided between the inner wall (5) and the outer wall (7).

2. The ventilation device (1) according to claim 1,
wherein
at least one stabilisation channel (9) is arranged in the intermediate lumen (8), wherein the hollow body element (4) can be brought into a second dimensionally stable state by supplying a predetermined amount of stabilisation fluid into the at least one stabilisation channel (9), wherein the stabilisation fluid can be supplied to and/or discharged from the at least one stabilisation channel (9) via at least one access device (10), wherein a fluidic connection exists between the at least one access device (10) and the at least one stabilisation channel (9) at a distal end (4a) of the hollow body element (4) , wherein the stabilisation fluid is air.

3. The ventilation device (1) according to claim 2,
wherein
the at least one access device (10) comprises at least one connection device (11) leading out of the ventilation device (1), wherein the at least one connection device (11) passes through an outer wall of the neck element (3), wherein the at least one access device comprises a valve device (12).

4. The ventilation device (1) according to one of claims 2 to 3,
wherein
the at least one stabilisation channel (9) has an extension which extends substantially from a distal end (4a) of the hollow body element (4), wherein the at least one stabilisation channel (9) extends substantially to a proximal end (4b) of the hollow body element (4), wherein the at least one stabilisation channel (9) extends substantially along a longitudinal direction (L) of the hollow body element (4) , wherein the at least one stabilisation channel (9) extends substantially rectilinearly or helically along the longitudinal direction (L) of the hollow body element (4), wherein the at least one stabilisation channel (9) ends in a proximal closure.

5. Ventilation device (1) according to one of claims 2 to 4,
wherein
at least one web element (13) is arranged between the inner wall (5) and the outer wall (7), wherein the at least one web element (13) extends essentially along a radial direction (R) of the hollow body element (4), wherein the at least one stabilisation channel (9) is formed by a section of the inner wall (5), a section of the outer wall (7) and the at least one web element (13).

6. The ventilation device (1) according to any one of claims 2 to 5,
wherein
between the inner wall (5) and the outer wall (7) at least one tubular element is provided, which forms the at least one stabilisation channel (9).

7. Ventilation device (1) according to one of the preceding claims,
wherein
the at least one intermediate layer is exposed between the inner wall (5) and/or the outer wall (7).

8. Ventilation device (1) according to one of the preceding claims,
wherein
a cuff (14) is arranged at a proximal end (4b) of the hollow body element (4), wherein the cuff (14) can be expanded to a predetermined size, wherein the expansion is effected by supplying an expansion fluid, wherein the cuff (14) is arranged on the inner wall (5) and/or the outer wall (7).

9. The ventilation device (1) according to claim 8,
wherein
the cuff (14) is fluidically connected to at least one stabilisation channel (9), wherein the expansion of the cuff (14) can be controlled via the at least one stabilisation channel (9), wherein a second access device (15) is provided by means of which the expansion of the cuff (14) can be controlled.

10. Ventilation device (1) according to one of the preceding claims,
wherein
the inner wall (5) and/or the outer wall (7) have at least one reinforcement section (25), wherein the inner wall (5) and/or the outer wall (7) have a reinforcement section (25) in the region of the adjacent cuff (14).

11. Ventilation device (1) according to one of the preceding claims,
wherein
a seal layer (16) is arranged on an inner surface (5a) of the inner wall (5), wherein a second intermediate lumen (17) is formed between the inner surface (5a) of the inner wall (5) and the seal layer (16), wherein the second intermediate lumen (17) is accessible via a third access device (18), wherein a negative pressure in the second intermediate lumen (17) is adjustable in a basic state

12. The ventilation device (1) according to claim 11,
wherein
in a closed state, a second expansion fluid can be introduced into the second intermediate lumen (17), whereby this expands, wherein the inner lumen (6) of the hollow body element (4) can be reduced by the expansion of the intermediate lumen.

## Revendications

1. Dispositif respiratoire (1), en particulier une canule trachéale (1a) destinée à être insérée dans un trachéostome, comportant un élément canule (2), lequel comporte un élément formant col (3) et un élément formant un corps creux (4) disposé sur l'élément formant col (3), l'élément formant un corps creux (4) présentant une paroi interne (5), laquelle entoure au moins une lumière interne (6), la paroi interne (5) étant entourée par une paroi externe (7), une lumière intermédiaire (8) étant définie entre la paroi interne (5) et la paroi externe (7), où, dans un premier état, l'élément formant un corps creux (4) est dans un état flexible, l'élément formant un corps creux (4) étant apte à être amené dans un deuxième état dimensionnellement stable par amenée d'une quantité prédéterminée de fluide de stabilisation dans la lumière intermédiaire (8), au moins une couche intermédiaire étant prévue entre la paroi interne (5) et la paroi externe (7).

2. Dispositif respiratoire (1) selon la revendication 1, dans lequel au moins un canal de stabilisation (9) est disposé dans la lumière intermédiaire (8), l'élément formant un corps creux (4) étant apte à être amené dans un deuxième état dimensionnellement stable par amenée d'une quantité prédéterminée de fluide de stabilisation dans ledit au moins un canal de stabilisation (9), le fluide de stabilisation étant apte à être amené audit au moins un canal de stabilisation (9) par l'intermédiaire d'au moins un dispositif d'accès (10) et/ou étant apte à être évacué dudit au moins un canal de stabilisation (9), une liaison fluidique se trouvant à une extrémité distale (4a) de l'élément formant un corps creux (4) entre ledit au moins un dispositif d'accès (10) et ledit au moins un canal de stabilisation (9), le fluide de stabilisation étant de l'air.

3. Dispositif respiratoire (1) selon la revendication 2, dans lequel ledit au moins un dispositif d'accès (10) comporte au moins un dispositif de liaison (11) sortant du dispositif respiratoire (1), ledit au moins un dispositif de liaison (11) traversant une paroi externe de l'élément formant col (3), ledit au moins un dispositif d'accès comportant un dispositif à soupape (12).

4. Dispositif respiratoire (1) selon l'une des revendications 2 et 3, dans lequel ledit au moins un canal de stabilisation (9) présente une étendue qui part sensiblement d'une extrémité distale (4a) de l'élément formant un corps creux (4), ledit au moins un canal de stabilisation (9) s'étendant sensiblement jusqu'à une extrémité proximale (4b) de l'élément formant un corps creux (4), ledit au moins un canal de stabilisation (9) s'étendant sensiblement le long d'une direction longitudinale (L) de l'élément formant un corps creux (4), ledit au moins un canal de stabilisation (9) s'étendant sensiblement en ligne droite ou en hélice le long de la direction longitudinale (L) de l'élément formant un corps creux (4), ledit au moins un canal de stabilisation (9) débouchant dans une fermeture proximale.

5. Dispositif respiratoire (1) selon l'une des revendications 2 à 4, dans lequel au moins un élément d'entretoise (13) est disposé entre la paroi interne (5) et la paroi externe (7), ledit au moins un élément d'entretoise (13) s'étendant sensiblement le long d'une direction radiale (R) de l'élément formant un corps creux (4), ledit au moins un canal de stabilisation (9) étant formé par une section de la paroi interne (5), une section de la paroi externe (7) et ledit au moins un élément d'entretoise (13).

6. Dispositif respiratoire (1) selon l'une des revendications 2 à 5, dans lequel, entre la paroi interne (5) et la paroi externe (7), au moins un élément tubulaire est prévu, lequel forme ledit au moins un canal de stabilisation (9).

7. Dispositif respiratoire (1) selon l'une des revendications précédentes, dans lequel ladite au moins une couche intermédiaire est exposée entre la paroi interne (5) et/ou la paroi externe (7).

8. Dispositif respiratoire (1) selon l'une des revendications précédentes, dans lequel une manchette (14) est disposée à une extrémité proximale (4b) de l'élément formant un corps creux (4), la manchette (14) étant expansible jusqu'à une taille prédéterminée, l'expansion s'effectuant par amenée d'un fluide d'expansion, la manchette (14) étant disposée sur la paroi interne (5) et/ou la paroi externe (7).

9. Dispositif respiratoire (1) selon la revendication 8, dans lequel la manchette (14) est en communication fluidique avec au moins un canal de stabilisation (9), un contrôle de l'expansion de la manchette (14) pouvant être effectué par l'intermédiaire dudit au moins un canal de stabilisation (9), un deuxième dispositif d'accès (15) étant prévu, au moyen duquel l'expansion de la manchette (14) peut être contrôlée.

10. Dispositif respiratoire (1) selon l'une des revendications précédentes, dans lequel la paroi interne (5) et/ou la paroi externe (7) présentent au moins une section de renforcement (25), la paroi interne (5) et/ou la paroi externe (7) présentant une section de renforcement (25) dans la zone de la manchette (14) adjacente.

11. Dispositif respiratoire (1) selon l'une des revendications précédentes, dans lequel une couche de fermeture (16) est disposée sur une surface interne (5a) de la paroi interne (5), une deuxième lumière intermédiaire (17) étant formée entre la surface interne (5a) de la paroi interne (5) et la couche de fermeture (16), la deuxième lumière intermédiaire (17) étant accessible par l'intermédiaire d'un troisième dispositif d'accès (18), une dépression pouvant être réglée dans un état de base dans la deuxième lumière intermédiaire (17).

12. Dispositif respiratoire (1) selon la revendication 11, dans lequel, dans un état de fermeture, un deuxième fluide d'expansion est apte à être introduit dans la deuxième lumière intermédiaire (17), ce qui provoque son expansion, l'expansion de la lumière intermédiaire permettant de réduire la lumière interne (6) de l'élément formant un corps creux (4).
